# EUROPÄISCHE PATENTSCHRIFT

(11) **EP 0 706 991 B1**
(45) Veröffentlichungstag und Bekanntmachung des Hinweises auf die Patenterteilung: **11.02.1998**
(21) Anmeldenummer: 95115501.9
(22) Anmeldetag: 30.09.1995
(51) Int. Cl.: C07C 67/60, C07C 69/54

(54) **Verfahren zur Herstellung von 1,4-Butandiolmono(meth)acrylat durch Veresterung von (Meth)acrylsäure mit 1,4-Butandiol, bei dem eine wässrige Lösung an nicht umgesetztem 1,4-Butandiol anfällt**
Process for the preparation of 1,4-butanediol mono(meth)acrylate by esterification of (meth)acrylic acid with 1,4-butanediol while obtaining an aqueous solution of non-converted 1,4-butanediol
Procédé de préparation du mono(méth)acrylate du 1,4-butanediol par estérification de l'acide (méth)acrylique avec le 1,4-butanediol avec obtention d'une solution aqueuse de 1,4-butanediol non-converti

(30) Priorität: 11.10.1994 DE 4436241
(43) Veröffentlichungstag der Anmeldung: 17.04.1996
(73) Patentinhaber: BASF AKTIENGESELLSCHAFT, 67056 Ludwigshafen (DE)
(72) Erfinder: Dockner, Toni, Dr., D-67149 Meckenheim (DE); Lermer, Helmut, Dr., D-67067 Ludwigshafen (DE); Bittins, Klaus, Dr., D-67227 Frankenthal (DE); Nestler, Gerhard, Dr., D-67061 Ludwigshafen (DE); Brauch, Gundo, Dr., D-67454 Hassloch (DE)

(56) Entgegenhaltungen:
- Keine einschlägigen Dokumente gefunden

## Beschreibung

Die vorliegende Erfindung betritt ein Verfahren zur Herstellung von 1,4-Butandiolmono(meth)acrylat durch Veresterung von (Meth)acrylsäure mit 1,4-Butandiol, bei dem eine wäßrige Lösung an nicht umgesetztem 1,4-Butandiol anfällt.

"(Meth)acrylsäure" wird als verkürzte Schreibweise verwendet und steht für Acrylsäure oder Methacrylsäure.

1,4-Butandiolmono(meth)acrylat ist bekannt (vgl. z.B. Ullmanns Encyclopädie der technischen Chemie, Polyacryl-Verbindungen bis Quecksilber, Bd. 19, 4. Auflage, Verlag Chemie (1980), S. 10). Aufgrund ihrer Bifunktionalität (Hydroxylgruppe und monoethylenisch ungesättigte Gruppe) stellen sie interessante Ausgangsverbindungen dar. Beispielsweise eignen sie sich als vinylisch ungesättigte Comonomere in z.B. als Bindemittel geeigneten, durch radikalische Polymerisation erzeugten Polymerisaten. Als alkoholische Verbindungen eignen sie sich aber auch z.B. für polymeranaloge Umsetzungen (z.B. Kondensations- oder Additionsreaktionen).

Es ist allgemein bekannt, 1,4-Butandiolmonoacrylat durch direkte Veresterung von (Meth)acrylsäure mit 1,4-Butandiol in Gegenwart saurer Veresterungskatalysatoren, unter Entfernung des entstehenden Wassers durch azeotrope Destillation mit einem geeigneten Schleppmittel, herzustellen.

Die Monoester entstehen dabei im wesentlichen nur dann in hohen Ausbeuten, wenn ein großer Überschuß des zweiwertigen Alkohols angewandt wird, da anderenfalls in nicht unerheblichen Mengen Diesterbildung einhergeht. Um ein solches Verfahren wirtschaftlich zu gestalten, ist es notwendig, diesen Überschuß zurückzugewinnen.

Nachteilig für eine solche Trennaufgabe ist, daß
- die Siedepunkte von Alkandiol, Monoester und Diester selbst unter reduziertem Druck bei erhöhten Temperaturen liegen;
- die Siedepunkte von Alkandiol, Monoester und Diester sehr nahe beieinander liegen;
- die vinylisch ungesättigten Monoester und Diester insbesondere in kondensierter Phase und bei erhöhter Temperatur eine erhöhte Polymerisationsneigung aufweisen.

Im Fall von Acrylsäure als Ausgangssäure betragen die relevanten Siedepunkte bei Normaldruck (1 atm) beispielsweise:
- 1,4-Butandiol: 230°C (Römpp Chemie Lexikon, 9. Auflage, Thieme Verlag, Stuttgart, 1989);
- 1,4-Butandiolmonoacrylat: ca. 230°C (Technische Information M/ED 1331 d der BASF Aktiengesellschaft aus 1987);
- 1,4-Butandioldiacrylat: ca. 225°C (Ullmanns Encyklopädie der technischen Chemie, Bd. 19, Verlag Chemie, Weinheim, 1980, S. 9).

Unter solchen Voraussetzungen scheidet eine rektifikative Auftrennung unter ökonomischen Gesichtspunkten faktisch aus, was so auch in der DE-A 42 28 397 auf Seite 1, Zeilen 35 bis 40, bestätigt wird.

Die DE-PS 15 18 572 und die EP-A 465 853 betreffen daher Verfahren zur Herstellung von 1,4-Butandiolmono(meth)acrylat durch direkte Veresterung von (Meth)acrylsäure mit 1,4-Butandiol in Gegenwart saurer Veresterungskatalysatoren, unter Entfernung des entstehenden Wassers durch azeotrope Destillation mit einem geeigneten Schleppmittel, die dadurch gekennzeichnet sind, daß bereits mit einer nur geringen relativen Einsatzmenge an 1,4-Butandiol ausgezeichnete Ausbeuten an 1,4-Butandiolmono(meth)acrylat erzielt werden. Charakteristisches Merkmal dieser Verfahrensweisen ist, daß aus dem Monoester, Diester und nicht umgesetztes 1,4-Butandiol enthaltenden Produktgemisch der Diester extraktiv abgetrennt und in die Veresterung rückgeführt wird, wobei die Verfahrensweise gemäß der EP-A 465 853 darüber hinaus die Veresterung im Beisein von erheblichen Mengen an zugesetztem vorgebildetem Diester ausführt.

Während die DE-PS 15 18 572 empfiehlt, die extraktive Abtrennung des Diesters im Beisein von Wasser durchzuführen, um zu verhindern, daß gleichzeitig nennenswerte Mengen an Monoester ins Extraktionsmittel übergehen, wird nach dem Verfahren der EP-A 465 853 die extraktive Abtrennung des Diesters in Abwesenheit einer wäßrigen Phase empfohlen, mit der Konsequenz, den bei dieser Extraktionsweise in das Extraktionsmittel übergehenden Monoester im nachhinein aus selbigem mit Wasser auswaschen zu müssen und die dabei anfallende wäßrige Phase mit der an Monoester reichen Extraktionsphase zu vereinigen.

D.h., sowohl gemäß der Verfahrensweise der DE-PS 15 18 572 als auch gemäß der EP-A 465 853 fällt im Verlauf der Herstellung eine wäßrige Lösung an, die neben 1,4-Butandiolmono(meth)acrylat nicht umgesetztes 1,4-Butandiol enthält.

Hinsichtlich dieser wäßrigen Phase wird nun in der EP-A 465 853 lediglich empfohlen, die darin enthaltenen leicht siedenden Bestandteile destillativ abzutrennen. Nachteilig an dieser Verfahrensweise ist, daß auf diese Weise lediglich ein mit 1,4-Butandiol verunreinigtes 1,4-Butandiolmono(meth)acrylat erhalten wird, das bezüglich zahlreicher Anwendungen nur geringe Brauchbarkeit aufweist. Beispielsweise können Alkandiole bei radikalischen Polymerisationen aufgrund ihrer regelnden Wirkung das gewünschte Molekulargewicht beeinträchtigen oder bei Umsetzung mit Diisocyanaten zu unerwünschter Vernetzung führen.

Die DE-PS 15 18 572 empfiehlt, das 1,4-Butandiolmono(meth)acrylat durch Extraktion mit einem geeigneten organischen Lösungsmittel aus der wäßrigen Lösung abzutrennen und anschließend dieses Extraktionsmittel destillativ zu entfernen. Hinsichtlich des Verbleibs des in der wäßrigen Lösung zurückbleibenden Wertproduktes 1,4-Butandiol schweigt sich die DE-PS 15 18 572 jedoch aus.

Die Aufgabe der vorliegenden Erfindung bestand daher darin, ein Verfahren zur Herstellung von 1,4-Butandiolmono(meth)acrylat durch Veresterung von (Meth)acrylsäure mit 1,4-Butandiol zur Verfügung zu stellen, bei dem in einfacher Weise eine effiziente Wertproduktabtrennung von in wäßriger Lösung anfallendem nicht umgesetztem 1,4-Butandiol durchgeführt wird.

Demgemäß wurde ein Verfahren zur Herstellung von 1,4-Butandiolmono(meth)acrylat durch Veresterung von (Meth)acrylsäure mit 1,4-Butandiol, bei dem eine wäßrige Lösung an nicht umgesetztem 1,4-Butandiol anfällt, gefunden, das dadurch gekennzeichnet ist, daß man das in der wäßrigen Lösung befindliche 1,4-Butandiol, vorzugsweise im Beisein katalytisch wirkender Mengen Säure, zu leicht flüchtigem Tetrahydrofuran umsetzt und das Tetrahydrofuran von der wäßrigen Lösung abtrennt. Vorzugsweise werden starke protische Säuren angewendet.

Tetrahydrofuran ist gemäß Ullmanns Encyklopädie der technischen Chemie, Bd. 12, Fungizide bis Holzwerkstoffe, Verlag Chemie, Weinheim, 4. Auflage (1976), S. 20ff, ein gesättigter cyclischer Ether, der bereits aufgrund seiner guten Lösungsmitteleigenschaften stark nachgefragt wird. So findet Tetrahydrofuran (THF) als solches Lösungsmittel ausgiebig in der Lack -und Folienindustrie Verwendung, sowie als Co-Lösungsmittel für Druckfarben und Klebstoffe. Gemäß vorgenannter Referenz beträgt der Siedepunkt von THF bei Normaldruck (1 atm) 66°C.

Aus der DE-PS 10 43 342 ist bekannt, daß THF durch katalytische Dehydratisierung von 1,4-Butandiol erhältlich ist. Aus derselben Schrift ist ferner bekannt, daß dabei häufig unerwünschte Nebenreaktionen auftreten und sämtliche für das Verfahren verwendeten Ausgangsstoffe maximal 30 % Wasser enthalten dürfen. Letzteres dürfte darauf zurückzuführen sein, daß die katalytische Dehydratisierung von 1,4-Butandiol zu THF reversibel ist. So wurde in der DE-PS 848 945 gefunden, daß man Diole erhält, wenn man tetrahydrierten 5-gliedrigen cyclischen Ether mit Wasser erhitzt und daß diese Umsetzung im Beisein saurer Mittel beschleunigt wird. Die Reversibilität der vorgenannten Dehydratisierung läßt sich auch dem Lehrbuch der organischen Chemie, C.R. Noller, Springer Verlag, Heidelberg (1960), auf S. 653 entnehmen.

Der Vorzug der erfindungsgemäßen Verfahrensweise liegt darin begründet, daß es sich bei THF um ein Wertprodukt handelt, dessen Siedepunkt signifikant unterhalb des Siedepunktes von 1,4-Butandiol liegt, so daß es aus wäßriger Lösung unter mäßigem Energieaufwand in einfacher Weise destillativ abgetrennt werden kann.

Als katalytisch wirksame Säuren kommen für die erfindungsgemäße Umsetzung beispielsweise Phosphorsäure, Schwefelsäure, p-Toluolsulfonsäure, Methansulfonsäure und entsprechende andere starke protische Säuren in Betracht. Bezogen auf die wäßrige Butandiollösung liegt ihr Gehalt in der Regel bei 0,1 bis 5, häufig bei 0,5 bis 2 Gew.-%.

In besonderer Weise eignet sich das erfindungsgemäße Verfahren zur Anwendung bei solchen wäßrigen nicht umgesetztes 1,4-Butandiol enthaltenden Lösungen, die im Rahmen der Veresterung von (Meth)acrylsäure mit 1,4-Butandiol zum Zwecke der Herstellung von 1,4-Butandiolmono(meth)acrylat unter nachfolgenden Verfahrensbedingungen anfallen (insbesondere dann, wenn als Ausgangssäure Acrylsäure verwendet wird):

Bei einer Temperatur von 80 bis 140°C werden in einer Kaskade aus 2 bis 4 Reaktionsgefäßen (Meth)acrylsäure und 1,4-Butandiol im Ausgangsmolverhältnis von 1:1 bis 1:3 in Gegenwart von 0,1 bis 3 Gew.-%, bezogen auf die zu veresternde Säure, saurem Katalysator (z.B. p-Toluolsulfonsäure, Schwefelsäure, Methansulfonsäure und Phosphorsäure) bei Normaldruck (1 atm) verestert, wobei das entstehende Wasser durch azeotrope Destillation mit einem Schleppmittel (ein aliphatischer oder cycloaliphatischer Kohlenwasserstoff, dessen Siedepunkt bei einem Druck von 1 atm 65 bis 120°C beträgt, vorzugsweise Cyclohexan) entfernt wird.

Die Menge des azeotropen Schleppmittels wird zweckmäßig so bemessen, daß der Reaktoraustrag aus dem letzten Gefäß der Kaskade 20 bis 40 Gew.-% Schleppmittel umfaßt.

Der Reaktoraustrag wird dem Mittelteil einer Extraktionskolonne (vorzugsweise einer Füllkörperkolonne mit Raschigringen) mit 2 bis 10, vorzugsweise 4 bis 6, theoretischen Trennstufen kontinuierlich zugeführt. Unter dem Mittelteil der Extraktionskolonne wird hier insbesondere der Teil verstanden, der von der Mitte des extraktiv wirksamen Teils (der extraktiv wirksamen Gesamtstrecke) der Extraktionskolonne aus betrachtet noch 50 % des extraktiv wirksamen oberen Teils und 50 % des extraktiv wirksamen unteren Teils der Extraktionskolonne umfaßt.

Am Kopf der Extraktionskolonne wird Wasser aufgegeben, während ins untere Ende der Kolonne das spezifisch leichtere Extraktionsmittel, das man mit dem für die Veresterung gewählten Schleppmittel chemisch identisch wählt, über eine Pulsationspumpe zugegeben wird. Die Extraktionstemperatur beträgt 20 bis 40°C. Das Extraktionsmittel, Extraktionsgut und das Wasser werden der Extraktionskolonne in solchen Gewichtsmengen zugeführt, daß gilt:
Gewicht zugeführtes Wasser / Gewicht zugeführtes Extraktionsgut = 2 bis 4, vorzugsweise 2 bis 3,
und
Gewicht zugeführtes Extraktionsmittel / Gewicht zugeführtes Extraktionsgut = 1 bis 4, vorzugsweise 2 bis 3.

Die am Kopf der Extraktionskolonne kontinuierlich zu entnehmende organische Phase enthält im wesentlichen nur 1,4-Butandiol(meth)acrylat gelöst und wird in die Veresterungsreaktion rückgeführt. Dabei erfolgt die Verteilung auf die verschiedenen Kaskadengefäße zweckmäßig entsprechend der aus diesen zu entfernenden Wassermenge.

Die dem unteren Ende der Extraktionskolonne kontinuierlich zu entnehmende wäßrige Phase enthält im wesentlichen 1,4-Butandiolmono(meth)acrylat und nicht umgesetztes 1,4-Butandiol.

Durch eine nachfolgende in an sich bekannter Weise durchzuführende Extraktion mittels Methylenchlorid, einem Ester aus C₁- bis C₄-Alkancarbonsäuren und C₁- bis C₅-Alkandiolen oder einem einfachen bzw. gemischten, 4 bis 8 C-Atome aufweisenden, Dialkylether wird aus der wäßrigen Phase 1,4-Butandiolmono(meth)acrylat abgetrennt. Vorzugsweise wird als Extraktionsmittel iso-Butylacetat angewandt. Durch einfache destillative Operation ist 1,4-Butandiolmono(meth)acrylat in hoher Reinheit erhältlich.

Die verbleibende wäßrige Lösung enthält in der Regel ≤ 10 Gew.-% an nicht umgesetztem 1,4-Butandiol (bezogen auf die Lösung).

Diese kann z.B. durch Eindampfen auf einen ≤ 30 Gew.-% betragenden Wassergehalt eingedampft und anschließend das darin enthaltene 1,4-Butandiol gemäß der in der DE-PS 10 43 342 empfohlenen Verfahrensweise in THF gewandelt werden, das in an sich bekannter Weise destillativ abgetrennt werden kann.

In überraschender Weise läßt sich die Wandlung des in der wäßrigen Lösung verbliebenen 1,4-Butandiol in THF aber auch ohne eine vorhergehende Aufkonzentrierung der wäßrigen Lösung mit auf 1,4-Butandiol bezogenen Umsätzen ≥ 90 Mol-% durchführen.

Vorzugsweise wird die Cyclisierung unter Eigendruck bei einer Temperatur von 140 bis 200°C im Beisein von 0,1 bis 5 Gew.- starker Säure durchgeführt.

Zweckmäßigerweise erfolgt die Umsetzung im Strömungsrohr bei Verweilzeiten von 10 bis 100 min. Vorzugsweise wird man die Reaktion in zwei hintereinander geschalteten Reaktionsrohren ausführen und in einem zwischengeschalteten Reaktionskessel durch Entspannen (vorzugsweise 1,5 atm, 110°C) einen Teil des gebildeten THF durch Strippen abtrennen. Aus dem Austrag des zweiten Reaktionsrohres wird das gebildete THF zweckmäßigerweise rektifikativ abgetrennt (üblicherweise P = 1 atm), wobei im oberen Teil der Kolonne vorzugsweise das im Rahmen der Zwischenstrippung anfallende wäßrige THF zusätzlich aufgegeben wird. Die Aufarbeitung des anfallenden THF/Wasser-Azeotrops erfolgt in an sich bekannter Weise. Mit Vorteil beträgt die Verweilzeit in den beiden hintereinander geschalteten Reaktionsrohren 15 bis 25 min (pro Reaktionsrohr).

Gemäß der vorstehend skizzierten Verfahrensweise sind im Rahmen der klassischen Veresterung von (Meth)acrylsäure mit 1,4-Butandiol zur Herstellung von 1,4-Butandiolmonoacrylat (Meth)acrylsäureumsätze ≥ 95 Mol-% bei gleichzeitigem 1,4-Butandiolumsatz von ≥ 99 Mol-% erreichbar. Dabei werden mehr als 98 Mol-% des eingesetzten 1,4-Butandiols in die Wertprodukte 1,4-Butandiolmono(meth)acrylat und THF gewandelt. Das Zielprodukt 1,4-Butandiolmono(meth)acrylat fällt in einer Reinheit ≥ 99 Gew.-% an.

Selbstverständlich erfolgen alle Verfahrensschritte auf dem Weg zum (Meth)acrylsäuremonoester im Beisein üblicher Mengen üblicher Polymerisationsinhibitoren. Als solche kommen z.B. Phenothiazin, Hydrochinon sowie Hydrochinonmonomethylether in Betracht.

### Beispiel

Eine 5 gew.-%ige Lösung von 1,4-Butandiol in Wasser wurde nach Zusatz von 1 Gew.-% konzentrierter Schwefelsäure (bezogen auf die Lösung) bei einer Verweilzeit von jeweils 20 min bei einer Temperatur von 180°C und einem Druck von 12,5 atm durch zwei hintereinander geschaltete Reaktionsrohre geführt. Nach Verlassen des ersten Reaktionsrohres erfolgte Zwischenentspannung auf 1,5 atm und 110°C, wobei gebildetes THF verdampft wurde. Der Reaktoraustrag wurde dann zusammen mit dem Brüden der Zwischenentspannung destilliert.

Es wurde ein Umsatz von 1,4-Butandiol zu THF von ≥ 95 Mol-% erzielt.

## Patentansprüche

1. Verfahren zur Herstellung von 1,4-Butandiolmono(meth)acrylat durch Veresterung von (Meth)acrylsäure mit 1,4-Butandiol, bei dem eine wäßrige Lösung an nicht umgesetztem 1,4-Butandiol anfällt, dadurch gekennzeichnet, daß man das in der wäßrigen Lösung befindliche 1,4-Butandiol zu Tetrahydrofuran umsetzt und das Tetrahydrofuran von der wäßrigen Lösung abtrennt.

2. Verfahren nach Anspruch 1, dadurch gekennzeichnet, daß man das in der wäßrigen Lösung befindliche 1,4-Butandiol im Beisein katalytisch wirkender Mengen Säure zu Tetrahydrofuran umsetzt.

3. Verfahren nach Anspruch 1 oder 2, dadurch gekennzeichnet, daß die anfallende wäßrige 1,4-Butandiollösung ≤ 10 Gew.-%, bezogen auf die Lösung, an 1,4-Butandiol enthält.

4. Verfahren nach Anspruch 1 bis 3, dadurch gekennzeichnet, daß die Umsetzung des in der wäßrigen Lösung befindlichen 1,4-Butandiol zu Tetrahydrofuran ohne vorherige Aufkonzentrierung derselben erfolgt.

5. Verfahren nach Anspruch 1 bis 4, dadurch gekennzeichnet, daß die Umsetzung von 1,4-Butandiol zu Tetrahydrofuran bei einer Temperatur von 140 bis 200°C erfolgt.

6. Verfahren nach Anspruch 1 bis 5, dadurch gekennzeichnet, daß die Umsetzung in einem Strömungsrohr bei einer Verweilzeit von 10 bis 100 min durchgeführt wird.

7. Verfahren nach Anspruch 1 bis 6, dadurch gekennzeichnet, daß es sich um ein Verfahren zur Herstellung von 1,4-Butandiolacrylat handelt.

## Claims

1. A process for the preparation of 1,4-butanediol mono(meth)acrylate by esterification of (meth)acrylic acid with 1,4-butanediol, in which an aqueous solution of unconverted 1,4-butanediol is obtained, wherein the 1,4-butanediol present in the aqueous solution is converted into tetrahydrofuran and the tetrahydrofuran is separated off from the aqueous solution.

2. A process as claimed in claim 1, wherein the 1,4-butanediol present in the aqueous solution is converted into tetrahydrofuran in the presence of a catalytic amount of an acid.

3. A process as claimed in claim 1 or 2, wherein the resulting aqueous 1,4-butanediol solution contains ≤ 10 % by weight, based on the solution, of 1,4-butanediol.

4. A process as claimed in any of claims 1 to 3, wherein the conversion of the 1,4-butanediol present in the aqueous solution into tetrahydrofuran is carried out without prior concentration of said solution.

5. A process as claimed in any of claims 1 to 4, wherein the conversion of the 1,4-butanediol into tetrahydrofuran is carried out at from 140 to 200°C.

6. A process as claimed in any of claims 1 to 5, wherein the reaction is carried out in a flow tube during a residence time of from 10 to 100 minutes.

7. A process as claimed in any of claims 1 to 6, which is a process for the preparation of 1,4-butanediol acrylate.

## Revendications

1. Procédé de préparation de mono(méth)acrylate de 1,4-butanediol par estérification d'acide (méth)acrylique avec du 1,4-butanediol, au cours duquel on obtient une solution aqueuse de 1,4-butanediol n'ayant pas réagi, caractérisé en ce que l'on transforme le 1,4-butanediol présent dans la solution aqueuse en tétrahydrofuranne et en ce que l'on extrait le tétrahydrofuranne de la solution aqueuse.

2. Procédé selon la revendication 1, caractérisé en ce que l'on transforme le 1,4-butanediol présent dans la solution aqueuse en tétrahydrofuranne, en présence de quantités catalytiques d'acide.

3. Procédé selon la revendication 1 ou 2, caractérisé en ce que la solution aqueuse de 1,4-butanediol contient 10% en poids ou moins de 1,4-butanediol, par rapport à la solution.

4. Procédé selon l'une quelconque des revendications 1 à 3, caractérisé en ce que l'on effectue la transformation du 1,4-butanediol présent dans la solution aqueuse en tétrahydrofuranne sans la concentrer au préalable.

5. Procédé selon l'une quelconque des revendications 1 à 4, caractérisé en ce que l'on effectue la transformation du 1,4-butanediol en tétrahydrofuranne à une température de 140 à 200°C.

6. Procédé selon l'une quelconque des revendications 1 à 5, caractérisé en ce que l'on effectue la transformation dans un réacteur tubulaire avec un temps de séjour de 10 à 100 min.

7. Procédé selon l'une quelconque des revendications 1 à 6, caractérisé en ce qu'il s'agit d'un procédé de préparation d'acrylate de 1,4-butanediol.
